# EUROPEAN PATENT APPLICATION

(11) **EP 4 082 528 A1**
(43) Date of publication of application: **02.11.2022**
(21) Application number: 21171612.1
(22) Date of filing: 30.04.2021
(51) Int. Cl.: A61K 9/06, A61K 47/10, A61K 9/12, A61K 31/00, A61K 47/08, A61K 47/12, A61K 47/14, A61K 47/20, A61K 47/32, A61K 47/38, A61P 31/12

(54) **LIQUID SPRAY FORMULATION**

(71) Applicant: GSK Consumer Healthcare SARL, 1260 Nyon (CH)
(72) Inventor: MÜLLER, Wolf-Dieter, 77815 Bühl (DE); STUBBS, Tim, Warwick CV34 4AB (GB)
(74) Representative: Haleon Patent Department

(57) **Abstract**

The present invention relates to a liquid formulation for spraying on a face mask, the formulation being an aqueous solution having a pH in a range between 2.5 and 4 and comprising an organic acid selected from a list consisting of citric acid, malic acid, lactic acid and tartaric acids, a film forming polymer, and an anionic surfactant. Other aspects of the invention are the use of such a liquid formulation to spray it on a face mask, and a method of forming a film on a face mask.

## Description

### FIELD OF THE INVENTION

The present invention relates to a sanitizing, anti-viral, anti-microbial liquid formulation comprising an anionic surfactant, an acid and a film forming polymer. The novel formulation is specifically designed for spray application on filter material suitable for a face mask.

### BACKGROUND TO THE INVENTION

PANDEMICS AND EPIDEMICS: In the past century five pandemics of respiratory infectious disease have been witnessed, of which the "Spanish flu" of 1918, caused by an A(H1N1) strain of influenza A virus, was the largest pandemics of any infectious disease known to medical science (Oxford, J.S., 2000). Excluding the current coronavirus disease (COVID-19) pandemic, strains of influenza belonging to group A have caused all of these pandemics (https://www.cdc.gov/flu/about/viruses/types.htm). The COVID-19 pandemic has spread from a novel coronavirus species that was observed as a human ARVI pathogen for the first time in December 2019 and later named severe acute respiratory syndrome coronavirus 2 (SARS-CoV-2). Human influenza A and B viruses also cause seasonal epidemics of respiratory disease known commonly as the flu season. A pandemic can occur for example when a new variant of influenza A virus or a novel virus, such as SARS-CoV-2, emerges that infects humans and has the ability to spread efficiently between people.

MECHANISM-OF-ACTION TRANSMISSION: Modes of infection and reproduction differ between the different families of respiratory viruses due to structural differences. Like other coronaviruses, SARS-CoV-2 particles are spherical and have four proteins; the spikes, membrane, envelope and nucleocapsid. The influenza A virion is also an enveloped virus, typically spherical, but sometimes filamentous, with glycoproteins hemagglutinin (HA) and neuraminidase (NA) forming spikes. An enveloped virus has its RNA is packaged within an outer lipid membrane. For replication and transmission of the enveloped virus, the lipid membrane containing the virus proteins needs to have the ability to release viral RNA whilst also providing structural stability to protect it. This renders the lipid membrane susceptible to soaps, many disinfectants, changes in temperature and pH. Non-enveloped viruses such as Rhinoviruses, Norovirus, or Poliovirus do not have such a lipid membrane, and are thus more resistant to certain stresses. In the case of Coronaviruses, to enable the release of viral RNA from the enveloped virus, the spike proteins latch onto human cells and undergo a structural change allowing the viral membrane to fuse with the cell membrane and the viral RNA to enter the host cell (NIH). Similar to the virus that caused the 2002 SARS outbreak, SARS-CoV-2 spikes bind to angiotensin-converting enzyme 2 (ACE2) receptors on the human cell surface. The SARS-CoV-2 spike is 10-20 times more likely to bind ACE2 on human cells than the spike from the 2002 SARS virus, enabling the SARS-CoV-2 to spread from human-to-human more efficiently.

CLINICAL SYMPTOMS: Both COVID-19 and influenza viruses have similar respiratory disease presentation. The clinical spectrum can range from asymptomatic through to severe acute respiratory infection (ARVI), sepsis with organ dysfunction and death. ARVI is an acute, often highly contagious, infection of the respiratory tract, present in the form of catarrhal inflammation of the upper respiratory airway, and progressing with fever, runny nose, sneezing, cough and sore throat.

PUBLIC HEALTH TRANSMISSION: The incubation period for a virus, which is the time between exposure to the virus and symptoms onset may vary. Transmissibility of the virus may depend on many factors not limited to viral load and symptoms, replication-competency of the virus, behaviour and environmental factors associated with the infected individual. (WHO) Liquid particles from an infected person's mouth or nose ranging in size from 'respiratory droplets' to smaller 'aerosols' can result in inhalation of, or inoculation with, the virus through the mouth, nose or eyes. This can occur through pre-symptomatic transmission (for people who are infected and shedding virus, but have not yet developed symptoms), asymptomatic transmission (from people infected who never develop symptoms) and symptomatic transmission (people infected with symptoms, high viral load and virus shedding).

CONTROL AND PREVENTION: Much has been done to control and prevent other pandemics from occurring with the development of many respiratory virus products such as anti-influenza treatments, diagnostics tests and vaccines. For those presently witnessing the COVID-19 pandemic, the limitations of these products in controlling the spread of SARS-CoV-2 are apparent with over 134 million cases globally (John Hopkins Coronavirus Resource Center, 7 Apr 2021). Currently available antiviral drugs for ARVI treatment are not optimal in controlling the spread of disease as the key molecules they target are effective against a specific virus family and species, which may vary due to different mechanisms (binding, fusion, DNA or RNA synthesis and replication, assembly and release of the virus), genotypes and expression of the target proteins. Innate or acquired mutation of different strains may further reduce efficacy of drug treatment. Preventative treatment with antiviral drugs is not favoured broadly due to potential side effects, drug resistance and high cost. Vaccine as part of a comprehensive prevention and control strategy are important. They may place a crucial part in preventing or minimising infection, but they do not always provide a transmission-blocking effect. Control of human-to-human transmission requires a comprehensive strategy that also comprises measures such as face mask use, physical distancing, keeping rooms ventilated, avoiding crowds, hand hygiene, respiratory etiquette, testing, contact tracing, quarantine and isolation. Face masks play a critical role in this strategy either by protecting healthy persons by filtering at inhalation of air or by preventing onward transmission by filtering at exhalation of air (source control).

Effectiveness of a face mask may be limited by their appropriate use, cleaning, storage or disposal along with the type (medical, FFP2, non-medical fabric), composition and quality of materials used. Essential parameters such as filtration, breathability and fit must be considered.

WO08009651 A1 discloses a pre-coated face mask with a novel oral and /or nasal air filter material capable of removing and neutralizing harmful virus from inhaled or exhaled contaminated air. The pre-coated face mask is obtained by dipping the substrate material for the mask into a loading solution, followed by a drying step. Such methods are very challenging to scale up to a commercially viable product due to the complicated manufacturing process and namely the technical difficulties in achieving a uniform coating. Furthermore, stability issues may occur with pre-coated masks, or more generally face-masks containing anti-viral actives, i.e. the anti-viral activity may decrease rapidly over time, depending on packaging and storage conditions.

There is thus an ongoing need to improve the effectiveness of face masks against transmission of viruses, in particular enveloped respiratory viruses such as coronaviruses (namely Sars-Cov-2) and the flu.

### SUMMARY OF THE INVENTION

According to a first aspect, the present invention is directed at a liquid formulation for spraying on a face mask, the formulation being an aqueous solution having a pH in a range between 2.5 and 4 and comprising an organic acid selected from a list consisting of citric acid, malic acid, lactic acid and tartaric acids, a film forming polymer, and an anionic surfactant.

For a formulation to be sprayable on a face mask it namely needs to have a suitable viscosity. Formulations with too high viscosity would require specific spraying equipment which cannot be integrated easily in a device for consumers. Furthermore, for a formulation to be suitable for spraying on a face mask, certain requirements in terms of toxicity and skin irritation must be fulfilled.

The formulation is sprayed on a conventional face mask , such as a surgical/medical mask, a FFP2 type mask, or a cotton mask by a consumer, and once sprayed on the mask, the polymer will form a hydrated matrix with the organic acid being dissolved therein. The film forming polymer provides the adhesion on the mask, and the organic acid is solubilised, so that no solid crystals are present which could be inhaled by the wearer.

The invention addresses several drawbacks of the prior art, namely of face masks which were pre-coated with anti-viral loading solutions or which contain anti-viral agents. Providing a liquid formulation to be sprayed on face mask to enhance its effectiveness in prevention of spread of a virus is very cost-effective. No separate manufacturing line is required, and production steps are reduced. Furthermore, the liquid formulation can be sprayed on the face mask prior to use, and when the user wears the face mask, the film formed on the mask will be fresh and remain intact and keep its efficacy for up to 12 hours, whereas pre-impregnated or pre-coated masks suffer from various shelf life issues. Finally, fitting of a face mask is personal, and the invention enables the user to choose the best fitting mask and use it in combination with the liquid formulation according to the invention.

A consumer can spray the liquid formulation on the outer surface of a face mask until the surface is entirely covered with the liquid formulation. The entire coverage of the mask surface with the liquid formulation having a pH of 2.5 to 3.5 ensures the same pH of 2.5 to 3.5 across the mask surface, and an even distribution of the formulation ingredients across the mask, which lead to the antiviral and antibacterial properties of the mask.

The low pH, obtained by the organic acid which gives away protons in the formulation, is one important factor contributing to the antiviral and antimicrobial efficacy of the liquid formulation. The antimicrobial effect is among others due to the prevention of absorption of essential nutrients by the microorganisms due to disruption of the protein motive force, which provides energy for active absorption of nutrients. This alters the permeability of cell wall causing damage and hence cell death, especially in gram negative bacteria. The organic acid reacts with virus-loaded-mucus droplets releasing hydrogen ions in high concentration. The hydrogen ions destroy the superficial enveloped virus membrane structure, precipitate proteins from the capsid and destroy the bonds of nucleic acids. The organic acid is also capable of chelating metal ions present in the cell wall thereby causing damage.

According to a preferred embodiment of the invention, the pH of the formulation is between 2.5 and 3.5, preferably between 2.7 and 3.3, more preferably between 2.9 and 3.2. The lower the pH, the higher is the antiviral and antimicrobial effect, but a too low pH leads to a corrosive effect which may damage the face mask tissue or irritate the skin if brought in contact therewith.

According to a preferred embodiment of the invention, the organic acid is citric acid. Both anhydrous and monohydrate citric acid can be used.

Citric acid is well known for being food safe, widely available, and odourless, and has a sufficient pka value to provide the desired acidity. Unpleasant odours such as the vinegar odour of acetic acid or pyruvic acids are not desirable for a liquid formulation for spraying on a face mask, since users might not want to be exposed to unpleasant odours while wearing a face mask. Furthermore, without wanting to be bound to a specific theory, the inventors believe that citric acid, which is known as a chelating agent, is capable of chelating metal ions present in the cell wall thereby causing damage and resulting in an antimicrobial / antiviral effect going beyond the effect linked to the low pH alone.

Preferably, anhydrous citric is present in a concentration of 5% to 20% (w/v), preferably 8% to 14% (w/v), more preferably 10% to 12% (w/v). The claimed concentrations ensure the desired efficacy, which is linked to the amount of citric acid present per mm² of the mask surface, without having to compromise on drying time.

According to a preferred embodiment of the invention, the liquid formulation further comprises a base forming a buffer system with the organic acid. Typically, the conjugate base of the organic acid used in the product can be added, for example sodium citrate if citric acid is used in the formulation. Alternatively, another base such as sodium hydroxide can be added, which then reacts with the organic acid to form its conjugated base. The addition of a sufficient amount of a base allows it to keep the pH of the formulation stable. If the formulation contains lactic acid, malic acid or tartaric acid, a corresponding conjugate base can be added, i.e. lactate, malate, or tartrate, or any base reacting with the acid to form the corresponding conjugate base.

According to a preferred embodiment, the film forming polymer is a hydrocolloid, more preferably selected from a list consisting of Polyvinylpyrrolidone (PVP), Polyvinyl alcohol (PVA), and Hydroxypropyl methylcellulose (HPMC). In principle, all edible or food-safe film forming polymers, namely hydrocolloids, could be used, and other options are hydroxypropyl cellulose (HPC), hydroxypropyl methylcellulose (HPMC), carboxymethylcellulose (CMC), methylcellulose (MC), starch alginate, carrageenan, chitosan, pectin, or xanthan gum.

Most preferably, the film forming polymer is Polyvinylpyrrolidone (PVP), also known as Povidone, Kollidon or Plasdone. Contrary to PVA and other film forming polymers which depending on the grade and the specific product may have an unpleasant odour, PVP is odourless, which makes it highly suitable for a use on a face mask, where odours are not desirable. Furthermore, PVP can absorb up to 40% of its weight in water, while not increasing the viscosity of the formulation significantly, and is thus an ideal choice for a formulation which is intended to form an even coating on a face mask when sprayed on. The high water absorption properties help to maintain a hydrated film on the face mask in the naturally humid environment to which a face mask is exposed when worn. This is an important safety aspect, since a desiccated, brittle film could present a risk of inhalation of labile solid particles.

Preferably a PVP with a K-value in aqueous solution of around 20 to 30, more preferably around 25, is used. A K-value of 25 corresponds to an approximate molecular weight of 30000, and this has been found to lead to optimal results in terms of sprayability and film properties on the mask. Furthermore, PVP, in particular PVP with a K-value as mentioned above, does not increase the viscosity of the formulation to an extent which would have a negative impact on the sprayability. The lower viscosity is also an advantage in manufacturing, as the PVP dissolves quickly. PVP with higher viscosity and higher molecular weight can act as a modified or delayed release matrix, and could thus inhibit the release of the organic acid, and thereby inhibit its availability of citric acid to provide viricidal effect.

According to a preferred embodiment of the invention, the film forming polymer is present in an amount of 2% to 4% (w/v), preferably in an amount of around 3%. Preferably, the liquid formulation contains 2% to 4% (w/v), most preferably around 3%, of PVP. It has been found that these amounts are sufficient to obtain a hydrated, evenly distributed film, and low enough to avoid any undesired stickiness of the formulation.

According to a preferred embodiment of the invention, the anionic surfactant is selected from a list consisting of sodium stearate, sodium cocosulfate, sodium laureth sulphate (SLES), ammonium lauryl sulphate, sodium myreth sulphate, and sodium lauryl sulphate (SLS).

More preferably, the anionic surfactant is sodium lauryl sulphate (SLS). Sodium lauryl sulphate (SLS) is an anionic surfactant with protein denaturing potency. The mechanism of action of SLS involves the solubilization of the viral envelope and/or the denaturation of envelope and/or capsid proteins, and SLS has thus a microbicidal effect on enveloped and non-enveloped viruses. SLS has also been shown to exert an antibacterial activity, which is one reason why it is included in some toothpaste products. The frequent use of SLS in products which are used on a daily basis such as toothpastes, mouthwashes, shampoos and liquid soaps suggest that this anionic surfactant could be used without having toxic effects on skin or mucosa, namely without causing skin irritation.

According to a preferred embodiment of the invention, the anionic surfactant is present in an amount of 0.2% to 0.6% (w/v), more preferably in an amount of 0.2 to 0.4% (w/v), most preferably in an amount of around 0.25%. According to a most preferred embodiment, the formulation contains between 0.2 to 0.3% (w/v) of SLS.

Experiments have shown that the desired efficacy is achieved with a SLS content of 0.25%, and while this was also the case for formulations with higher SLS content, a lower SLS content is preferred for safety and cost reasons. Even more importantly, experiments have shown that a higher SLS contents lead to higher drying times of the liquid formulation on the mask. An important objective of the present invention has been to provide a formulation which dries quickly once it has been sprayed on the face mask by a user, so that the face mask can be worn shortly after the formulation has been sprayed on the mask. Optimal drying time requires that a critical amount of the composition be absorbed by the filter material, penetrates in sufficient quantities and dries within an appropriate time frame for the patient/user to apply the face mask. This is in particular challenging when the formulation does not contain ethanol, isopropyl alcohol, or other alcohols in significant quantities, but contains mainly water as the solvent. Ethanol, isopropyl alcohol (isopropanol), and other alcohols evaporate more quickly, and would thus lead to shorter drying times, but are not desirable because the lead to corrosion of the mask material, and thus alter the filtering properties of the mask, as experiments carried out have shown. Furthermore, they are not desirable because of the health risks associated with their ingestion or the inhalation of vapours, because of the risk of inflammation. The experiments carried out clearly showed that a SLS content between 0.2% and 0.4% (w/v) is the optimum in terms of efficacy and drying time for water-based formulation

According to a preferred embodiment, the liquid formulation has a viscosity below 5 mm²/s, preferably below 5 mm²/s, and most preferably around 1mm²/s, i.e. close to the viscosity of water, thereby ensuring good sprayability with simple spray nozzles. Higher viscosity formulations require aerosol sprays or other more sophisticated spray devices for delivery, which lead to a more expensive and less sustainable end product.

According to a preferred embodiment, the formulation contains no ethanol, isopropyl alcohol (isopropanol) or other alcohols in substantial quantities. Preferably, only water is used as the solvent, for the reasons already indicated above.

According to a preferred embodiment, the formulation further comprises edetate disodium (EDTA), preferably in an amount between 0.1 and 0.2% (w/v). EDTA provides additional microbial stability to the formulation.

According to another aspect of the invention, the invention relates to a metered-dose pump spray product, containing a liquid formulation as previously described. A metered-dose spray pump allows for easy spraying of the formulation on a face mask, and precise dosing instructions (number of spray activations) can be given, leading to optimal results.

According to another aspect of the invention, the liquid formulation as previously described is used to spray it on a face mask.

According to yet another aspect of the invention, the invention relates to a method of forming a film on a mask comprising the steps of spraying a liquid as previously described on a face mask. Preferably, the face mask is a medical face mask, a FFP2 face mask, or a cotton mask with at least 3 layers of tissue.

Preferably, the film forming polymer forms a hydrated polymer matrix on the face mask, with the organic acid being present in an amount of around 0.4 to 1.5 mg/cm², preferably 0.4 to 0.9mg/cm².

### DESCRI PTI ON OF DRAWINGS/ FIGURES

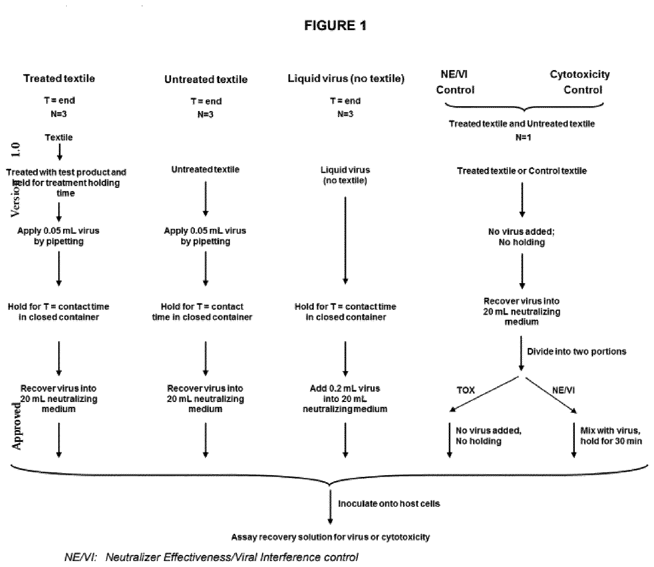

FIG. 1 shows a flow chart illustrating experiments which were carried out to test efficacy.

### DETAILED DESCRIPTION OF THE INVENTION

### EXAMPLES

Table 1 shows five examples of liquid formulations according to the invention

**Table 1**

| | **Example 1** | **Example 2** | **Example 3** | **Example 4** | **Example 5** |
|---|---|---|---|---|---|
| **Ingredient** | **%w/v** | **%w/v** | **%w/v** | **%w/v** | **%w/v** |
| Citric Acid (anhydrous) | 10.97 | 10.97 | 10.97 | 3 | 10.97 |
| Sodium Lauryl Sulphate | 0.25 | 0.25 | 0.25 | 0.25 | 0.375 |
| Polyvinyl Pyrrolidone K25 | 3.00 | 3.00 | 3.00 | 3.00 | 3.00 |
| Disodium Edetate | 0.0166 | 0.0166 | 0.0166 | 0.0166 | 0.0166 |
| Sodium Hydroxide | 1.55*) | 1.7**) | 2.7***) | *) | 2.7*) |
| Purified Water | q.s. to 100 | q.s. to 100 | q.s. to 100 | q.s. to 100 | q.s. to 100 |

| | | | | | |
|---|---|---|---|---|---|
| *) quantity to reach pH 3.0 **) quantity to reach pH 3.3 ***) quantity to reach 3.6 | | | | | |

To prepare the formulations, the ingredients were weighed out, and 80% of water was added to a mixing vessel. The mixer was turned on to create a vortex. Anhydrous citric acid was added and mixed until dissolved. Sodium hydroxide pellets were added until dissolved. Disodium edetate (EDTA) was added and mixed until dissolved. Polyvinyl Pyrrolidone K25 (PVP) was slowly added to the vortex and mixed until dissolved and until a clear solution was obtained. Sodium Lauryl Sulphate (SLS) was added and mixed until dissolved, with mixing speed being reduced to minimize foaming. Once a clear solution had been obtained, water was added to obtain the desired volume , and it was mixed again for a homogenous product. The solution was then filtered through a 25-micron filter and filled in HDPE bottles with a metered dose pump.

A comparative example with 40% ethanol (instead of water, example not shown in table 1) was also prepared and tested. The tests showed that due to the alcohol present in the formulation, for all face masks except for 100% cotton masks, the mask tissue did not remain intact, but deteriorated. The use of formulations containing ethanol or isopropanol was therefore discarded.

### VIRUCIDAL EFFICACY TEST ("VET")

VET was conducted to evaluate the virucidal effectiveness of the formulation of Example 1 against SARS-associated Coronavirus Type 2 (SARS-CoV-2) when applied to various textile products. VET was designed to simulate consumer use and was based on the International Standard ISO 18184 method "Textiles - Determination of Antiviral Activity of Textile Products". The VET protocol is illustrated in FIG. 1 and will be discussed in more detail below.

In summary, samples of three types of textile products (Substrate 1, Substrate 2, and Substrate 3) were treated with the formulation of Example 1, then held for one of two holding times, then treated with SARS-CoV-2, then held for one of four exposure periods, then the surviving virus was recovered, diluted, and inoculated onto a cell host to determine the amount of remaining infectious virus units. Three replicates (N=3) were performed.

The viral inoculum for all tests and control was prepared using SARS-CoV-2, strain USA-WA1/2020 (source: BEI Resources, Catalog no.: NR-52281). The host cell line used was Vero E6 cells (source: ATCC, Catalog no.: CRL-1586).

The textile products were exposed to UV light under a biosafety cabinet for a minimum of 15 minutes per side and 2.5 cm x 2.5 cm pieces were cut from the textile products to obtain the test substrates. Substrate 1 was obtained from a surgical mask (unifree 3-ply level 1 facemask with ear loops). Substrate 2 was obtained from a cotton mask (100% double layer facemask). Substrate 3 was obtained from a FFP2 class filtering mask (Coast KN95 Safety Mask).
The substrates were placed in a clean, sterile petri dish with the outer layer facing up. A spray bottle was placed about 10-15 cm above the substrates at a 45-degree angle. Each substrate was sprayed with 1 spray and then moved to another dry and clean petri dish for the treatment holding time in a biosafety cabinet.

After the treatment holding time, the substrates were then treated with 0.05 mL (5 drops of 10 microliters) of viral inoculum by pipetting, added in multiple spots and distributed evenly across the substrate. The inoculated substrates were then placed in a 50 mL conical tube and capped. The inoculated substrates were held in the capped tube for the contact time.

After completion of the contact time, 20 mL of a neutralizer (viral recovery medium) was added to the tube and vortexed five times for at least 5 seconds each time to extract the virus. After the vortex, the substrates were manually squeezed against the inner wall of the tube using a cell scraper to remove additional liquid from the substrate.

The extracted liquid was centrifuged at 1000 rpm for 3 minutes to remove debris from the substrate and supernatant collected. An aliquot was then be ten-fold serially diluted in dilution medium, inoculated into host cells, and the inoculated host cells incubated at 36 +/- 2 degree C with 5 +/- 3% CO2 for about 4-9 days. The residual infectious virus in all test and control samples were detected in the incubated host cells by viral induced cytopathic effect (CPE) and scored by examining all test and control samples.

The 50% tissue culture infectious dose per mL (TCID50/mL) were determined using the method of Spearman-Karber or other appropriate methods such as Reed and Muench, Am. J. of Hyg. 1938, 27:493. In the case where a sample contained no detectable virus, a statistical analysis was performed based on Poisson distribution to determine the theoretical maximum possible titer for that sample. The results were reported as the reduction of the virus titer due to treatment with test material expressed as log10.

One untreated control of each type of textile product was tested in parallel to the treated textile products to serve as the Virus Recovery Control. In addition, a liquid virus control without any textile was performed using the same amount of viral inoculum to serve as the Virus Input Control.

Additional controls were carried out, and showed that:
1) None of the treated or control textile-extraction solutions did exhibit any cytotoxicity.
2) The difference in viral titer (Log10 TCID50/mL) between the treated textile NE/VI sample and the control textile NE/VI sample, or between the treated textile NE/VI sample and the theoretical titer was ≤ 0.5 Log10.
3) No virus was detected in the Cell Viability Control.
4) The cells remain viable throughout the course of the assay period. In addition, it confirms the sterility of the media employed throughout the assay period

The results of the Virucidal Efficacy Tests are provided in Table 2 below.

**Table 2**

| **Test Substance** | **PostTreatment Holding Time** | **Viral Contact Time** | **Initial Load (Log¹⁰ TCID⁵⁰)*** | **Output Load (Log¹⁰ TCID⁵⁰)**** | **Log¹⁰ Reduction** |
|---|---|---|---|---|---|
| Substrate 1 | 20 Minutes | 3 minutes | 5.77 | 4.35 | 4.73 |
| | | 5 minutes | | ≤ 2.42 | ≥3.35 |
| | | 10 minutes | | ≤ 2.13 | ≥3.64 |
| | | 30 minutes | | ≤ 2.13 | ≥3.64 |
| | 12 hours | 3 minutes | | 4.35 | 1.42 |
| | | 5 minutes | | 4.27 | 1.50 |
| | | 10 minutes | | ≤ 2.34 | ≥3.43 |
| | | 30 minutes | | ≤ 2.13 | ≥3.64 |
| Substrate 2 | 20 Minutes | 3 minutes | 5.44 | 3.85 | 1.59 |
| | | 5 minutes | | 3.43 | 2.01 |
| | | 10 minutes | | 2.29 | 3.15 |
| | | 30 minutes | | ≤ 2.13 | ≥3.31 |
| | 12 hours | 3 minutes | | 4.02 | 1.42 |
| | | 5 minutes | | 3.69 | 1.75 |
| | | 10 minutes | | 2.97 | 2.47 |
| | | 30 minutes | | ≤ 2.23 | ≥3.21 |
| Substrate 3 | 20 Minutes | 3 minutes | 5.64 | 4.60 | 1.04 |
| | | 5 minutes | | 4.44 | 1.20 |
| | | 10 minutes | | 4.35 | 1.29 |
| | | 30 minutes | | ≤ 2.34 | ≥3.30 |
| | 12 hours | 3 minutes | | 4.27 | 1.37 |
| | | 5 minutes | | 4.31 | 1.33 |
| | | 10 minutes | | 3.73 | 1.92 |
| | | 30 minutes | | ≤ 2.12 | ≥3.51 |
| * Input load is derived from the average of the three replicates of the control fabric. The control fabric was used as the input load control as a worst-case scenario. | | | | | |
| ** Output load is derived from the average of the three replicates of the treated fabric. | | | | | |
| ≥ Denotes a complete inactivation of the virus challenged. | | | | | |

Log R> 3.0 was obtained within a 10 min contact time after a product application of either 20 min or 12 hours on Substrate 1. Log R>3.0 was obtained within a 30 min contact time after a product application of either 20 min or 12 hours on Substrate 2. Log R>3.0 was obtained within a 10 min contact time after a product application of 20 min and after 30 min contact time after a product application of 12 hours on Substrate 3.

It was observed that treated mask substrate material with one spray (delivering approximately 100µL) of the liquid formulation of Example 1 resulted in a significant reduction in viral titres (>99.9%) for all mask material types (cotton, FFP2 or medical masks). The product has shown to be efficacious within a 10 to 30 min contact time depending on the mask type. The efficacy remained for at least up to 12 hours after product application.

### SURFACE pH MEASUREMENTS ON TREATED FACE MASKS

Further experiments as described below were performed to investigate the anti-viral efficacy of the liquid formulation of Examples 1, 2, and 3 when sprayed on an entire mask (medical, cotton, FFP2). The entire mask, as opposed to the 2.5cm X 2.5 cm sample used in the VET experiments above, were treated with the same liquid solution of Example 1 at a pH of 2.6, Example 2 at a pH of 3.3, and Example 3 at a pH of 3.6.

A first set of each mask type (medical, FFP2, cotton) was treated with 6 sprays of the liquid solution of Example 1, a second set of each mask type was treated with 6 sprays of the liquid solution of Example 2, and a third set of each mask type was treated with 6 sprays of the liquid solution of Example 3. Per spray puff, around 100µl of the liquid formulation were delivered.

The pH was measured by acid colorphast pH indicating strips. The pH was consistent across the surface of each mask of each set, with no significant differences being observed between different areas of the mask. The pH measurements also showed no significant change in pH on mask surfaces after a 20 min, 60 min, and a 20 hour drying time, staying within a range of about 3.3 to about 3.6 for medical/FFP2 mask types and a range of 3.6 to 3.9 for the cotton mask type. Therefore, it can be expected that the antiviral effect will also be consistent across the mask surface, and that it will remain present for at least the tested time periods.

It was also observed that dilution of 60 microliters of the liquid solution of Example 1 on a 1x1 sample of a medical mask type with 100 microliters and 200 microliters of water did not reduce surface pH when similar volumes of the liquid solution of Example 1 were applied to the mask.

### DRYING TIME

The amount of evaporation of moisture from the antiviral spray after application onto mask surfaces (open/unfolded) was evaluated over time. The liquid formulation of Example 1 was applied (12, 10, or 9 sprays, delivering around 100µl per spray puff) onto the surface of different mask types. Weight loss was observed over time along with appearance and wetness noted at each time point. The experiment was terminated once the weight plateaued. Results showed that greater than 50% weight loss was observed after 20 minutes drying for all mask substrate material types, and only minimal further weight loss was observed after 40 minutes drying for all mask types.

### RESIDUE TRANSFER

To further explore drying time needed for recommendations on product labels, the amount of transfer of coating residue from mask surfaces was quantified. Each mask surface was treated with 12 sprays of the liquid formulation of Example 1. The mask surface was pinched using Kimwipe swatch (2in x 0.75in) between two fingers to simulate adjusting the mask. The wipe was weighed to measure the residue picked up from the mask. This data was used to extrapolate the amount of citric acid that is available to be transferred fingers. After 20 minutes of drying, the average residue transferred from each mask surface was reduced by at least 95%.

### VOLATILITY STUDY

Medical masks were treated with 12 sprays of the liquid solution of Example 1 and left for 20 or 45 minutes drying time. Blank masks with no spray were also evaluated. The mask was then rolled and placed into a flask and sealed with a septum. The headspace was then extracted for 10 minutes using a 1cm Divinylbenzene/Carboxen/Polydimethylsiloxane (DVB/CAR/PDMS) Solid Phase Microextraction (SPME) fiber. The SPME fiber was then injected into the Gas chromatography-Mass spectrometry (GCMS) instrument containing a 30mX0.250mmX0.25-micron DB5 column using helium as the carrier gas with the following program: constant pressure, 35° C held for 3 minutes; heat 4° C per minute to 200° C; heat 20° C per minute to 280° C. Results showed that the relative amount of volatiles from the spray were not significantly different (0.1% volatile peak area) at 20 minutes compared to 45 minutes drying time. There were more volatiles from the mask surface than from the spray. In some cases, the spray may suppress the volatility of some compounds.

### DEPTH OF PENETRATION STUDY

A Scanning Electronic Microscope using the Energy Dispersive X-Ray (SEM-EDX) was used to visualize the liquid solution of Example 1 and to understand the depth of penetration of the product. FFP2 CE certified mask, cotton mask with two layers (100% cotton), and 3-ply polypropylene medical mask surfaces were treated with 12 sprays of mask solution. The samples were left to dry for 24 hours. Elevated sodium was used to indicate the mask solution presence when comparing untreated and treated mask layers. Results showed treated cotton masks had a minor amount of mask solution coating on the inner surface. As a precaution, it is recommended to use this product on cotton masks with at least three layers to mitigate risk of potential exposure to skin. For medical masks, results showed an insignificant amount of spray coating present on the inner layer, and therefore no exposure to the skin. For FFP2 masks, no mask solution made it to the middle layer or inner layer and therefore does not present exposure of the product to the face.

### DYNAMIC VAPOR SORPTION STUDY

A small quantity of either dehydrated mask solution (the solution having a solution similar to Example 1 except that enough NaOH was added to obtain a pH of 3.5) or powdered ingredients comprising the formula (replacing water and NaOH with sodium citrate) were placed onto a 13mm quartz plate. A dynamic vapor sorption (DVS) ramp was begun at 25°C. The dehydrated mask solution (which exists as a thin, soft, clear film) began to take up additional moisture starting at 50% relative humidity and gained up to approximately 13% moisture at 90% humidity, 25 degrees C. Since the breath provides 100% humidity, the formulation of Example 1 is believed to exist as a hydrated polymer matrix adhered to mask fibers, in which citric acid is dissolved.

### ADHESION OF MASK SOLUTION TO MASK SURFACES AS COATING

After application of the spray solution onto the mask surface, the coating becomes fixed to mask fibers. A polymer matrix of PVP is believed to form a clear film that binds citric acid and other formula ingredients to mask fibers. A thin film coats fibers after application and the product remains partially hydrated during use. This implies maintenance of adhesion of the coating and solubilized citric acid to the mask during use. It can be concluded that as citric acid is solubilized, no solid crystals are present to be aerosolized into the wearer's breath.

### STABILITY

A formulation according to Example 1 was stored for 4 months, and the content in citric acid and SLS were measured after at the beginning and after the 4 months of storage. The citric acid content and the SLS content had not significantly decreased, and no microbial contamination was detected. The formulation was thus considered stable. A formulation according to Example 1, but additionally containing 0.5% sodium benzoate, also underwent the same stability testing, and no difference between the formulations with and without sodium benzoate could be found.

## Claims

1. Liquid formulation for spraying on a face mask, the formulation being an aqueous solution having a pH in a range between 2.5 and 4 and comprising
a. an organic acid selected from a list consisting of citric acid, malic acid, lactic acid and tartaric acids,
b. a film forming polymer, and
c. an anionic surfactant.

2. Liquid formulation according to claim 1, **characterised in that** the pH of the formulation is between 2.5 and 3.5, preferably between 2.7 and 3.3, more preferably between 2.9 and 3.2.

3. Liquid formulation according to claim 1 or 2, **characterised in that** the acid is citric acid.

4. Liquid formulation according to claim 3, **characterised in that** anhydrous citric is present in a concentration of 5% to 20% (w/v), preferably 8% to 14% (w/v), more preferably 10% to 12% (w/v).

5. Liquid formulation according to any of the preceding claims, **characterised in that** it contains a base forming a buffer system with the organic acid.

6. Liquid formulation according to any of the preceding claims, **characterised in that** the film forming polymer is a hydrocolloid.

7. Liquid formulation according to claim 6, **characterised in that** the film forming polymer is a hydrocolloid selected from a list consisting of Polyvinylpyrrolidone (PVP), Polyvinyl alcohol (PVA), and Hydroxypropyl methylcellulose (HPMC).

8. Liquid formulation according to claim 7, **characterised in that** the film forming polymer is Polyvinylpyrrolidone (PVP).

9. Liquid formulation according to any of the preceding claims, **characterised in that** the film forming polymer is present in an amount of 1% to 4% (w/v), preferably in an amount of around 3%.

10. Liquid formulation according to any of the preceding claims, **characterised in that** the anionic surfactant is selected from a list consisting of sodium stearate, sodium cocosulfate, sodium laureth sulphate (SLES), ammonium lauryl sulphate, sodium myreth sulphate, and sodium lauryl sulphate (SLS).

11. Liquid formulation according to claim 8, **characterised in that** the anionic surfactant is sodium lauryl sulphate (SLS).

12. Liquid formulation according to any of the preceding claims , **characterised in that** the anionic surfactant is present in an amount of 0.1% to 0.6%, preferably in an amount of 0.2% to 0.4%, most preferably in an amount of around 0.25%.

13. Liquid formulation according to any of the preceding claims, **characterised in that** it has a viscosity below 5 mm²/s, preferably around 1mm²/s.

14. Liquid formulation according to any of the preceding claims , **characterised in that** it further comprises edetate disodium (EDTA), preferably in an amount between 0.1 and 0.2% (w/v).

15. Liquid formulation according to any of the preceding claims, **characterised in that** it does not comprise any substantial amounts of alcohols, namely no ethanol and no isopropyl alcohol.

16. Metered-dose pump spray product, containing a liquid formulation according to any of the preceding claims.

17. Use of a liquid formulation according to any of the preceding claims to spray on a face mask.

18. Use of a liquid formulation according to claim 17, wherein the face mask is a surgical mask, a FFP2 mask, or a cotton mask with at least 3 layers.

19. Method of forming a film on a mask comprising the steps of spraying a liquid formulation according to any of claims 1 to 15 on a face mask.

20. Method according to claim 19, wherein the film forming polymer forms a hydrated polymer matrix, with the organic acid being present in an amount of around 0.4 to 1.5 mg/cm² on the face mask surface, preferably in an amount of around 0.4 to 0.9 mg/cm².

21. Face mask which comprises a film layer formed by spraying with a liquid formulation according to any of claims 1 to 15, wherein the organic acid is present in an amount of around 0.4 to 0.9 mg/cm² on the face mask surface.
